# EUROPÄISCHE PATENTANMELDUNG

(11) **EP 2 418 061 A1**
(43) Veröffentlichungstag der Anmeldung: **15.02.2012**
(21) Anmeldenummer: 11008886.1
(22) Anmeldetag: 20.11.2009
(51) Int. Cl.: B29C 39/02, B29C 39/10, B29C 67/00

(54) **Lasersinterpulver und Verfahren zur Identifizierung von Lasersinterpulvern und daraus hergestellten Objekten**

(30) Priorität: 20.11.2008 DE 102008058177; 16.12.2008 US 201832 P
(62) Teilanmeldung aus: 09806002.3
(71) Anmelder: EOS GmbH Electro Optical Systems, 82152 Krailling (DE)
(72) Erfinder: EOS GmbH Electro Optical Systems, 82152 Krailling (DE)

(57) **Zusammenfassung**

Es wird ein Pulver vorgestellt, das als Baumaterial in einem Schichtbauverfahren, wie beispielsweise einem selektiven Lasersinterverfahren, verwendet werden kann und dem mindestens ein Salz eines Metalls der seltenen Erden beigemischt ist, wobei das Salz die Eigenschaft aufweist, dass es eine Lumineszenz zeigt, wenn es mit Photonen einer Wellenlänge außerhalb des sichtbaren Spektrums oder mit Teilchenstrahlung bestrahlt wird. Dadurch können das Pulver und die mittels des Schichtbauverfahrens aus dem Pulver erzeugten Bauteile bezüglich des Herstellers, Herstellungsortes oder Herstellungsdatums identifiziert werden.

## Beschreibung

Die Erfindung ist auf ein Verfahren gerichtet, das es erlaubt, die bei Schichtbauverfahren verwendeten pulverförmigen Ausgangs-Stoffe hinsichtlich ihrer Herkunft zu kennzeichnen, sowie auf die Verwendung solch eines gekennzeichneten Pulvers als Ausgangsmaterial in einem Schichtbauverfahren.

Bei der schichtweisen Herstellung von Objekten aus pulverförmigen Ausgangsstoffen beispielsweise mittels Lasersintern stellt sich das Problem, dass die pulverförmigen Ausgangsstoffe in ihrem Aussehen nicht zu unterscheiden sind, obwohl es sich um unterschiedliche Pulver handelt.

Beispielsweise kann einem Pulver optional ein Flammschutzmittel zugesetzt sein, um eine Feuerbeständigkeit des zu erzeugenden Objektes zu erreichen. Solch ein Zusatz verändert in der Regel nicht das Aussehen des Pulvers. Ob ein Flammschutzmittel zugesetzt wurde, kann dann in der Regel nur durch eine aufwendige Analyse des Pulvers festgestellt werden.

Des weiteren kann es auch wichtig sein, bei einem bereits hergestellten Objekt bzw. Bauteil festzustellen, aus welchem Pulver es gefertigt wurde. Dies kann z.B. Teil einer Fehleranalyse sein, wenn das Objekt nicht die gewünschten Eigenschaften aufweist.

Selbst wenn das Objekt einwandfrei ist, kann es z.B. erwünscht sein, manchmal nach Jahren, den Lieferanten des verwendeten Lasersinterpulvers zu kennen. Hierbei soll bei einer Analyse in der Regel das Objekt nicht beschädigt werden müssen, beispielsweise wenn eine Probe entnommen wird.

Ein Zusatz von Farbpigmenten zu dem Pulver zur Kennzeichnung unterschiedlicher Pulvereigenschaften ist ungeeignet, da dadurch die Endfarbe der zu erzeugenden Objekte beeinflusst wird, was in vielen Fällen unerwünscht ist.

Aus dem Stand der Technik (WO2006/119759) ist bekannt, als zusätzlichen Bestandteil einen Markierungsstoff in Schüttgüter oder Kunststoffe einzubringen, um daraus gefertigte Produkte von einem Plagiat unterschieden zu können. Entsprechend wird ein Produkt in seiner Gesamtheit markiert. Nachteilig bei dem bekannten Stand der Technik ist der hohe Analyseaufwand da die Kosten einer Analyse in der Regel die Kosten des markierten Produktes übersteigen.

Die Aufgabe der Erfindung ist es daher, ein Pulver bereit zustellen, das einfach identifizierbar und/oder markierbar ist und insbesondere geeignet ist in einem Lasersinterverfahren Bauteile herzustellen, ohne dass das Aussehen des Pulvers und/oder der Bauteile durch die Identifizierbarkeit und/oder Markierbarkeit verändert wird.

Die Überlegungen, die zur Lösung der Aufgabe führten machten sich die Erkenntnis zu eigen, dass die Existenz von beigemischten lumineszierenden Markierungs- und/oder Identifizierungsstoffen, in vorteilhafter Weise berührungslos und kostengünstig durch eine optische Strahlung außerhalb des sichtbaren Spektrums detektierbar ist und diese Beimischung das Aussehen des Pulvers und/oder der Bauteile im sichtbaren Licht nicht verändert.

Durch das erfindungsgemäße Verfahren ist es insbesondere möglich, sowohl die Pulver zu identifizieren als auch die aus dem Pulver gefertigten Bauteile exakt einem bestimmten verwendeten Ausgangspulver zuzuordnen.

Damit kann auch noch nach vielen Jahren, wenn Aufzeichnungen verloren gegangen sind oder Lieferketten nicht mehr lückenlos nachverfolgbar sind, festgestellt werden, aus welchem Ausgangsmaterial welchen Herstellers die entsprechenden Teils erzeugt wurden. Insbesondere ist es auch möglich, dass das Pulver nicht nur entsprechend einem bestimmten Hersteller bzw. Herstelldatum gekennzeichnet wird, sondern dass auch ein Hinweis auf denjenigen angebracht wird, der mit diesem Pulver Bauteile erzeugt hat. Durch das erfindungsgemäße Verfahren ist es des weiteren möglich, eine Identifizierung selbst dann vorzunehmen,- wenn nur beliebige, kleine Bruchstücke von Bauteilen vorliegen.

Weitere Merkmale und Zweckmäßigkeiten der Erfindung werden im Folgenden anhand eines Ausführungsbeispiels beschrieben.

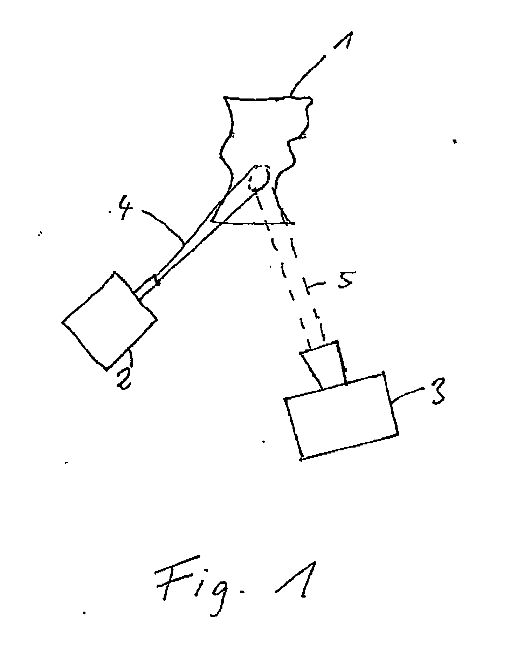

Fig. 1 zeigt einen beispielhaften Aufbau zur Veranschaulichung eines Verfahrens zur Untersuchung eines gekennzeichneten Pulvers oder daraus hergestellten Bauteils.

Zur Kennzeichnung eines in einem Schichtbauverfahren, beispielsweise einem Lasersinterverfahren, als Baumaterial verwendbaren Pulvers dergestalt , dass dessen Eigenschaften oder dessen Herkunft von einem speziellen Hersteller erkennbar sind, wird gemäß der Erfindung das Pulver in einem handelsüblichen Mischer mit einem Markierungspulver vermischt. Damit eine zu starke Änderung der Eigenschaften des Ausgangspulvers durch das Markierungspulver vermieden wird, ist es günstig, wenn der Anteil des Markierungspulvers an der Mischung einen gewissen Prozentsatz, z.B. 20 Gew.-%, nicht überschreitet. Natürlich ist ein noch geringerer Anteil, beispielsweise 10 Gew.-%, oder besser noch zwischen 0.1 und 10 Gew.-%, noch vorteilhafter. Allerdings sinkt mit dem Anteil des Markierungspulvers auch die Wahrscheinlichkeit, noch in kleinen Proben der Gesamtmischung Markierungsstoffpartikel zu finden.

Im Hinblick auf eine Pulveridentifizierung auch bei kleinen Pulvermengen ist es auch wichtig, dass die Vermischung der einzelnen Bestandteile so vollständig stattfindet, dass das Endprodukt möglichst homogen ist.

Da die Eigenschaften der herzustellenden Objekte durch die Hinzufügung des als Identifikationsmittel dienenden Markierungsstoffes nicht verändert werden sollen , muss der Markierungsstoff in sichtbarem Licht farblos sein, oder aber in so einem geringen Mengenverhältnis hinzugefügt sein, dass keine Veränderung der Farbe des Ausgangspulvers erkennbar ist.

Um dennoch ein Pulver oder Bauteilidentifizieren zu können, wird erfindungsgemäß als Markierungsstoff und/oder Identifizierungsstoff eine Substanz gewählt, die eine Lumineszenz zeigt , wenn sie mit Licht einer Wellenlänge außerhalb des sichtbaren Bereichs, beispielsweise IR-Licht oder UV-Licht bestrahlt wird. Für die Identifizierung muss dann das von dem lumineszierenden Stoff emittierte Licht auf seine Wellenlänge und/oder Intensität hin untersucht werden.

Dadurch kann allein anhand des Vorliegens einer Lumineszenzemission bestimmt werden, ob ein Markierungsstoff dem Pulver beigesetzt wurde. Falls eine Lumineszenzemission bei Bestrahlung festzustellen ist, kann anhand der eingestrahlten (anregenden) und/oder der emittierten Wellenlänge(n) ermittelt werden, welcher Markierungsstoff zugesetzt wurde. Ein Pulver kann nun dadurch gekennzeichnet werden, dass ein Markierungsstoff zugesetzt wird, welcher eine ganz bestimmte Wellenlänge oder mehrere charakteristische Wellenlängen oder einen bestimmten Wellenlängenbereich emittiert.

Natürlich ist es auch möglich, einen Markierungsstoff zu verwenden, der eine Lumineszenz in verschiedenen Wellenlängenbereichen zeigt. Allgemein ist eine Identifizierung über die Registrierung einer ganz bestimmten Spektralverteilung im emittierten Licht möglich.

Das erfindungsgemäß markierte Pulver kann als Baumaterial in einem beliebigen Schichtbauverfahren zum Herstellen von dreidimensionalen Objekten verwendet werden, also z.B. in einem selektiven Sinter- oder Schmelzverfahren unter Anwendung von Laserstrahlung oder Elektronenstrahlung oder Infrarotstrahlung oder aber einem 3D-Druckverfahren, bei dem zum Verfestigen des Materials ein Bindemittel aufgespritzt wird. Die genannten Verfahren sind unter anderem in WO 90/03893 und US 6,375,874 B 1 beschrieben.

Ein beispielhafter Aufbau zur Untersuchung eines mittels markiertem Pulver hergestellten Bauteils ist in Fig. 1 dargestellt.

Auf ein lasergesintertes Bauteil 1 wird dabei mit Hilfe einer UV-Lichtquelle 2 UV-Licht 4 gestrahlt. Ein Teil des durch die Bestrahlung angeregten Lumineszenzlichtes 5 wird durch einen Detektor 3 erfasst.

Alternativ zu UV-Licht kann auch Licht mit einer anderen Wellenlänge außerhalb des sichtbaren Bereichs, z.B. im IR-Bereich, in einer bevorzugten Ausbildung der Erfindung im nahen Infrarot (NIR), noch bevorzugter zwischen 900 nm und 1000 nm, für die Anregung der Lumineszenz verwendet werden. Des Weiteren ist auch eine Anregung der Lumineszenz mittels ionisierender Strahlung (Teilchenstrahlung oder Röntgenstrahlung) möglich. Der Detektor für die Untersuchung des Lumineszenzlichts kann dabei eine einfache Photodiode oder aber auch ein CCD oder Pixelsensor sein, der die Lichtmenge erfasst.

Der Nachweis der Anwesenheit eines Markierstoffes geschieht im einfachsten Fall durch Vergleich der Lichtemissionen des Pulvers oder fertiggestellten Bauteils mit und ohne Anregungslicht. Die Identifizierung der Wellenlängen des Lumineszenzlichts kann z.B. durch Filtervorsätze vor dem Detektor realisiert werden, wobei die Filtervorsätze jeweils nur eine Transmission in einem begrenzten Wellenlängenbereich zeigen. Denkbar ist aber auch die Verwendung anderer Aufbauten, die eine Spektralzerlegung liefern (z.B. Prismen, Gitter, etc.). Die Spektralzerlegung kann dabei auch im Detektor selbst stattfinden.

Zur Bestimmung der Konzentration an Markierungsstoffen im Pulver bzw. Bauteil wird beispielsweise die emittierte Menge an Lumineszenzlicht einmal ohne vorgesetztes Filter und zum anderen mit einem vorgesetzten Filter gemessen, wobei das Filter nur im Bereich des Lumineszenzspektrums eine Transmission zeigt. Auf diese Weise kann die Menge des Lumineszenzlichts in Relation zu der insgesamt vom Pulver oder Bauteil reflektierten Lichtmenge, die auf den Detektor fällt, gesetzt werden. Unter der Voraussetzung einer geeigneten Kalibrierung des Systems kann man dann die Menge des zugesetzten Markierstoffs bestimmen, falls es die zugesetzte Markierstoffmenge ist, die zur Kodierung von Information verwendet wird. In einer abgewandelten Ausführungsform werden dem Pulver zwei verschiedene Markierungsstoffe zugesetzt, die bei der Lumineszenz eine Lichtemission in verschiedenen Wellenlängenbereichen zeigen und/oder verschiedene anregende Wellenlängen besitzen.

Eine bestimmte Codierung kann dann erzeugt werden durch Bemessen des Mengenverhältnisses der beiden zugesetzten Markiersubstanzen zueinander. Das Mengenverhältnis wird dann bei der Untersuchung des Pulvers oder Bauteils dadurch ermittelt, dass die in den beiden unterschiedlichen Wellenlängenbereichen emittierten Lichtmengen zueinander in Relation gesetzt werden. Auf diese Weise kann eine entsprechende Codierung gelesen werden. Natürlich können auch mehr als zwei unterschiedliche Markiersubstanzen zugesetzt werden. Des Weiteren ist es auch möglich, einen Markierungsstoff zu verwenden, der eine Lumineszenz in verschiedenen Wellenlängenbereichen zeigt.

Selbst wenn die Emissionsbereiche zweier Markiersubstanzen einander überlappen, können bei Verwendung eines Spektrometers zur Analyse der Lumineszenzstrahlung dennoch die relativen Mengenverhältnisse der beiden Substanzen ermittelt werden.

Das beschriebene Verfahren lässt sich auf alle möglichen Pulver anwenden, also insbesondere auf Polymerpulver, Metallpulver und Sandsinterpulver.

Da für einige der genannten Pulver sehr hohe Temperaturen beispielsweise während eines Sinter - oder Schmelzprozesses auftreten, ist eine wichtige Anforderung an die Auswahl der Markierstoffe die, dass die Markiersubstanzen durch die hohen Temperaturen, die während des Bauvorgangs auftreten, nicht beeinträchtigt werden. Es wurde gefunden, dass bei Verwendung von Salzen der seltenen Erden eine Temperaturbeständigkeit für die normalerweise bei Lasersinter Verfahren auftretenden Temperaturen gegeben ist. Hierzu zählen z.B. Oxide der seltenen Erden oder Oxi- Sulfide oder auch Fluoride, die mit kleinen Beimengungen anderer Elemente, die ebenfalls der Gruppe de seltenen Erden entstammen, dotiert sind, um die gewünschte Lumineszenz zu erzeugen.

Die Markiersubstanz kann in besonders vorteilhafter Weise so zugemischt werden, dass die Teilchen der Markiersubstanz auf der Oberfläche der Pulverteilchen eingebettet werden. Auf diese Weise kann man jedes einzelne Pulverteilchen markieren. Hierzu werden die Markierungssubstanz und die Pulverteilchen beispielsweise einem in der EP 0 555 947 A1 beschriebenen Verfahren für die Oberflächenbehandlung von Teilchen unterzogen. Dabei werden die Partikel einer von mehreren, untereinander verbundenen Aufschlagkammern zugeführt, die mit einer Drehscheibe mit Aufschlagstiften versehen sind, sowie mit einem Aufprallring , so dass das Gemisch einem Schlagprallvorgang ausgesetzt wird, wodurch ein durch diesen Vorgang erzeugter Luftstrom von dem Pulvergemisch getrennt und kontinuierlich aus der Aufschlagkammer freigegeben wird, wobei der Aufschlagvorgang wiederholt wird und das Pulvergemisch vorübergehend in der Aufschlagkammer ruhen kann, bevor es in die darauffolgende Kammer weitergeleitet wird.

### Versuche mit einer solchen handelsüblichen

Pulverbehandlungsmaschine NHS-1 der Firma Nara ergaben, dass die Behandlungsdauer bei einer Umdrehungszahl von 8.000 U/min mindestens eine Minute betragen muss (bei Raumtemperatur).

Es soll abschließend noch betont werden, dass man unterschiedliche Markiersubstanzen nicht nur daran erkennen kann, welches Licht nach einer Anregung emittiert wird, sondern auch daran, durch welche Wellenlänge(n) die Lumineszenz angeregt wird.

## Patentansprüche

1. Pulver, welches als Baumaterial in einem Schichtbauverfahren verwendet werden kann, wobei
- das Pulver aus verschiedenen Bestandteilen unterschiedlicher Stoffe besteht,
- eines oder mehrere der Bestandteile des Pulvers ein für Markierungs- und/oder Identifizierungszwecken verwendbares Salz eines Metalls der seltenen Erden ist,
**dadurch gekennzeichnet, dass**
- das oder die Salze bei Bestrahlung mit Photonen einer Wellenlänge außerhalb des sichtbaren Spektrums oder bei Bestrahlung mit Teilchenstrahlung die Eigenschaft hat Lumineszenz zu zeigen und
- das oder die Salze in Abhängigkeit der Eigenschaften des Pulvers und/oder in Abhängigkeit von der Identität des Pulverherstellers und/oder der Identität des Anwenders und/oder in Abhängigkeit vom Herstellungsort des mittels des Schichtbauverfahrens hergestellten Bauteils gewählt sind.

2. Pulver nach Anspruch 1,
**dadurch gekennzeichnet, dass**
in dem Pulver Bestandteile aus einem oder mehreren unterschiedlichen Salzen der seltenen Erden vorhanden sind, deren Lumineszenz ein jeweils unterschiedliches Spektrum aufweisen.

3. Pulver nach Anspruch 1 oder 2,
**dadurch gekennzeichnet, dass**
das Pulver durch die Auswertung der bei Bestrahlung mit Photonen einer Wellenlänge außerhalb des sichtbaren Spektrums oder bei Bestrahlung mit Teilchenstrahlung auftretenden Lumineszenz identifizierbar ist.

4. Pulver nach einem der Ansprüche 1 bis 3,
**dadurch gekennzeichnet, dass**
dotierte Oxide, Oxi-Sulfide oder auch Fluoride der seltenen Erden Bestandteile Pulvers sind.

5. Pulver nach Anspruche 4,
**dadurch gekennzeichnet, dass**,
Yttriumoxid (Y.0)-, Yttriumoxisulfid (Y₂0₂S) oder Natrium-Yttrium-Flourid (NaYF)., jeweils dotiert mit Erbium oder einem anderen Element der seltenen Erden, Bestandteile des Pulvers sind.

6. Pulver nach einem der Ansprüche 1-5
**dadurch gekennzeichnet, dass**
der Anteil der zugefügten Salze am Pulver zwischen 0.1 und 10 Gew.-% liegt.

7. Pulver nach einem der Ansprüche 1-6
**dadurch gekennzeichnet, dass**
das Pulvers eine homogene Mischung seiner Bestandteile ist.

8. Verfahren zur Herstellung eines Pulvers nach einem der Ansprüche 1 bis 7,
**dadurch gekennzeichnet, dass**
die Salze durch scherendes Mischen mit dem Pulver vermischt werden.

9. Verfahren zur Herstellung eines Pulvers nach einem der Ansprüche 1 bis 7,
**dadurch gekennzeichnet, dass**
das oder die Salze mit dem Pulver dergestalt vermischt werden, dass die Pulverpartikel einem Schlagprallvorgang ausgesetzt werden, der dazu führt, dass das Salz auf der Oberfläche der Pulverteilchen eingebettet wird.

10. Verwendung eines Pulver nach einem der Ansprüche 1-7,
**dadurch gekennzeichnet, dass**
die Codierung durch Auswahl und/oder Kombination und/oder Menge eines bestimmten Salzes oder mehrerer bestimmter Salze geschieht.

11. Verwendung eines Pulvers nach einem der Ansprüche 1 bis 7 als Baumaterial in einem Verfahren zur Herstellung eines dreidimensionalen Objektes durch schichtweises Aufbringen und Verfestigen eines pulverförmigen Aufbaumaterials an den dem Querschnitt des herzustellenden Objektes in der jeweiligen Schicht entsprechenden Stellen , wobei die Verfestigung durch Einwirkung eines Lasers oder einer anderen Energiequelle auf das Pulvermaterial oder durch selektives Aufbringen eines Bindemittels geschieht.

12. Verwendung eines Pulvers nach Anspruch 11,
**dadurch gekennzeichnet, dass**
das Verfahren zur Herstellung des dreidimensionalen Objektes ein selektives Lasersinterverfahren oder ein selektives Laserschmelzverfahren ist.
